# EUROPÄISCHE PATENTSCHRIFT

(11) **EP 1 800 662 B1**
(45) Veröffentlichungstag und Bekanntmachung des Hinweises auf die Patenterteilung: **16.04.2008**
(21) Anmeldenummer: 06026551.9
(22) Anmeldetag: 21.12.2006
(51) Int. Cl.: A61K 8/36, A61K 8/92, A61Q 19/08

(54) **Wirkstoffkombination aus Santalbinsäure oder Derivate davon und Traubenkernöl zur topischen oder dermatologischen Verwendung**
Active ingredient combinations comprising santalbic acid and grape seed oil for topical and dermatological use
Combinaisons d'ingrédients actives contenant d'acide santalbinique et d'huile de pépins de raisin pour une utilisation topique ou dermatologique

(30) Priorität: 21.12.2005 DE 102005061071
(43) Veröffentlichungstag der Anmeldung: 27.06.2007
(73) Patentinhaber: Kneipp-Werke Kneipp-Mittel-Zentrale GmbH & CO. KG, 97082 Würzburg (DE)
(72) Erfinder: Wohlfart, Rainer Dr., 97230 Sulzfeld (DE); Naumann, Angela, 97537 Wipfeld (DE); Dauphin, Claudia, 97222 Rimpar (DE); Weymann, Andrea, 97264 Eibelstadt (DE)
(74) Vertreter: Langöhrig, Angelika Beate

(56) Entgegenhaltungen:
- EP-A1- 0 304 603
- WO-A-02/098436
- FR-A1- 2 633 616
- US-A1- 2005 158 258
- BOMBARDELLI E ET AL: "MICROVASCULOKINETIC ACTIVITY OF XIMENYNIC ACID ETHYL ESTER" FITOTERAPIA, IDB HOLDING, MILAN, IT, Bd. 65, Nr. 3, 1994, Seiten 195-201, XP001104828 ISSN: 0367-326X

## Beschreibung

Die vorliegende Erfindung betrifft Wirkstoffkombinationen und deren kosmetische Zusammensetzungen, bestehend aus Monoacetylenfettsäuren mit mindestens einer Doppelbindung, vorzugsweise Santalbinsäure oder Derivate davon und Traubenkernöl zur Pflege und zum Schutze der Haut, insbesondere der empfindlichen Haut, vorzugsweise der durch intrinsische und/oder extrinsische Faktoren gealterten oder alternden Haut sowie die Verwendung dieser Wirkstoffkombinationen auf dem Gebiet der kosmetischen und dermatologischen Hautpflege, insbesondere deren Verwendung zur Prophylaxe und Behandlung von trockener /atopischer Haut des Menschen und ferner zur Behandlung von Bindegewebsschwäche und Cellulite.

Unter kosmetischer Hautpflege ist in erster Linie zu verstehen, dass die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) gestärkt oder wiederhergestellt wird. Die Haut übt als das größte Organ des Menschen zahlreiche lebenswichtige Funktionen aus. Sie schützt z.B. vor Kälte, Hitze, Strahlung, dem Einwirken chemischer Substanzen und Krankheitserregern. Sofern die Haut diese Barrierefunktion nicht mehr ausreichend erfüllt, können lokale Irritationen oder auch ganzkörperliche Beschwerden entstehen.

Die Haut, insbesondere die Epidermis, ist als Barriereorgan des menschlichen Organismus in besonderem Maße äußeren Einwirkungen unterworfen. Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedene polare Lipide werden im Keratinisierungsprozess freigesetzt.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Bei alternder Haut beispielsweise erfolgt die regenerative Erneuerung verlangsamt, wobei insbesondere das Wasserbindungsvermögen der Hornschicht nachlässt. Sie wird deshalb inflexibel, trocken und rissig ("physiologisch" trockene Haut). Ein Barriereschaden ist die Folge. Die Haut wird daher anfällig für negative Umwelteinflüsse wie die Invasion von Mikroorganismen, Toxinen und Allergenen. Infolge dessen kann es zu toxischen oder allergischen Hautreaktionen kommen.

In einem optimalen Zustand der Haut liegt ein ausgewogenes Verhältnis von Hautlipiden und Hautfeuchtigkeit vor. Durch dieses Gleichgewicht werden wichtige Eigenschaften der Haut, wie das Penetrationsvermögen, Wasserbindungsvermögen, Elastizität, Regenerationsfähigkeit oder die Widerstandsfähigkeit gegen Umwelteinflüsse und Noxen unterschiedlichster Art, bestimmt. Den Hautlipiden, besonders den Oberflächenlipiden, ist dabei eine übergeordnete Bedeutung zuzuschreiben.

Der äußere Lipidfilm der Haut stellt eine sehr komplex zusammengesetzte Emulsion aus verschiedenen Lipiden und dem Sekret der Schweißdrüsen, wie Harnstoff, Fettsäuren, anorganische Salze und Wasser, dar. Die Bestandteile der Ölphase stammen vorrangig aus den Absonderungen der Talgdrüsen, die u.a. Squalen, Cholesterol und Cholesterolester, Ester vom Wachstyp, Triglyceride und freie Fettsäuren enthalten.

Die Regulation des Wasser- und Feuchtigkeitsgehaltes ist eine der wichtigsten Funktionen der epidermalen Lipidmembran. Allerdings hat sie nicht nur eine Barrierewirkung gegen externe chemische und physikalische Einflüsse, sondern trägt auch zum Zusammenhalt der Hornschicht bei. Die Zusammensetzung dieser Lipide, die über die gesamte Hornschicht verteilt vorliegen, ist für die intakte Funktion der epidermalen Barriere und damit für die Regulation des Wasserhaushalts der Haut von entscheidender Bedeutung.

Bei trockener oder atopisch veränderter Haut kommt es zu einer Verringerung dieser Hautlipide. Damit einhergehend kommt es zu einer Verringerung der Hautfeuchtigkeit und einer Erhöhung des transepidermalen Wasserverlustes.

Traubenkernöl (*Vitis Vinifera, Grape seed oil*) ist als Basis für Speiseöl und kosmetische Präparate bekannt.

Im Stand der Technik ist ferner die kosmetische Verwendung von Santalbinsäure (CAS 557-58-4 bzw. trans-11-Octadecen-9-ynoic acid, Santalbic acid, Ximeninic acid, Ximenynic acid, 11-Octadecen-9-ynoic acid, (E)- (8CI9CI)) bekannt (FR 2 633 516, EP 0 304 603).

Santalbinsäure ist Bestandteil des Sandelbaums und wird zur therapeutischen Behandlung von Ekzemen eingesetzt. Fisher (US 2005/0158258A1) beschreibt eine kosmetische Zusammensetzung bestehend aus Santalbinsäure, Niacin, Alpha-Liponsäure sowie Pilzextrakten. Traubenkernöl ist als Nebenbestandteil und zwar als Feuchtigkeitsmittel beschrieben.

Es ist Aufgabe der vorliegenden Erfindung neue Wirkstoffkombinationen bereitzustellen, die den komplexen Anforderungen an einer kosmetischen Zusammensetzung zur topischen oder dermatologischen Verwendung gerecht wird, insbesondere zur Pflege der Haut geeignet ist und die oben beschriebenen Anforderungen umfassend erfüllt.

Die Aufgabe wird für den Fachmann nicht vorhersehbar gelöst durch eine Wirkstoffkombination bestehend im Wesentlichen aus einem primären Wirkstoff und zwar Traubenkernöl und einem zweiten Wirkstoff nämlich monoacetylierten Fettsäuren enthaltend mindestens eine weitere Doppelbindung und zwar Verbindungen der Formel (I) bestehend aus mindestens 12 Kohlenstoffatomen: 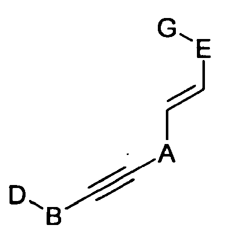 mit A eine Bindung oder (CH₂)ₙ mit n= 1-6
und B und E unabhängig voneinander C₂-C₁₂ Alkylen, C₂-C₁₂ Alkenylen,
D und G beide COR oder zumindest D oder G COR, wobei der jeweils andere H, CH₃ ist und R unabhängig voneinander für D oder G ausgewählt ist aus der Gruppe OH, CH₃, C₂H₅, NH₂.

Bevorzugt sind Verbindungen mit A = eine Bindung oder A = CH₂ und n=1.

Ferner bevorzugt ist B und E mit (CH₂)ₙ und n= 4-8 und D oder G = COR, wobei der jeweils andere CH₃ ist und R OH oder CH₃ ist.

Formel (I) umfasst ebenfalls beliebige entsprechende Stereoisomere, wie E (trans) und Z (cis) Isomere oder Salze, wie z.B. das Natriumsalz der entsprechenden Carbonsäuren.

Ganz besonders bevorzugt ist Santalbinsäure oder dessen Ester.

Überraschender Weise weisen diese erfindungsgemäßen Wirkstoffkombinationen einen hautstraffenden Effekt auf und zwar dergestalt, dass bereits nach kurzzeitiger Einwirkung in die Haut die periphere Hautblutzirkulation, bzw. die Hautdurchblutung erhöht wird. Dies hat eine unmittelbare Wirkung auf die Hautgeschmeidigkeit bzw. Hautstraffheit. Hierbei ist beachtlich, dass Traubenkernöl ausgezeichnete Eigenschaften als Gleitmittel aufweist und beim Einmassieren der erfindungsgemäßen Wirkstoffkombination zu einem positiven Effekt führt.

Ferner weist die erfindungsgemäße Wirkstoffkombination einen zusätzlichen curativen Effekt auf, so dass Cellulite wirksam behandelt werden kann. In einer weiteren Ausführungsform ist daher die erfindungsgemäße Wirkstoffkombination zur Prophylaxe und Behandlung von Bindegewebsschwäche sowie Cellulite (Celluliten, Orangenhaut) geeignet.

Ferner werden hinsichtlich der Hautfeuchtigkeit signifikante Effekte bewirkt, die unmittelbar auf die erfindungsgemäße Wirkstoffkombination zurückzuführen sind. Eine besonders vorteilhafte Wirkung ist darin zu sehen, dass Schlackenstoffe und Fette im Hautsystem besser abgeführt werden.

Die erfindungsgemäße Wirkstoffkombination, insbesondere in der Ausgestaltung einer kosmetischen Zusammensetzung, weist eine Vielzahl von Vorteilen auf und vereint verschiedene technische Effekte (Steigerung der Hautdurchblutung, Gleitmittel mit einhergehendem Massageeffekt, Wirkung gegen Cellulite, Erhöhung der Hautfeuchtigkeit, Hautgeschmeidigkeit, resultierende Hautstraffung) die zu einer überlegenen kosmetischen Wirkung führen.

Besonders bevorzugt sind daher solche Zusammensetzungen einer Wirkstoffphase, die die erfindungsgemäße Wirkstoffkombination in folgenden Verhältnissen enthält: Traubenkernöl in Gew % 1-99%, vorzugsweise 1-90 %, 1-50%, 1-20% und eine Verbindung bestehend aus mindestens 12 C-Atomen gemäß Formel (I) in Gew % 0,01 - 5, vorzugsweise Gew % 0,02-1, vorzugsweise Gew % 0,02-0,5. Ferner können diese Zusammensetzungen weitere Zusatzstoffe und / oder Hilfsstoffe enthalten und betreffen daher kosmetische Zusammensetzungen.

In einer besonders bevorzugten Ausführungsform der Erfindung sind wasserfreie Zusammensetzungen enthaltend die erfindungsgemäße Wirkstoffkombination bevorzugt. Eine solche wasserfreie Zusammensetzung ist nicht abschließend ein entsprechendes Hautöl enthaltend die erfindungsgemäßen Wirkstoffkombination.

Im Rahmen dieser Erfindung wird unter Traubenkernöl, das aus den Traubenkernen durch Pressung / kalte oder heiße Extraktion und anschließender Raffination (Entsäuern, Bleichen, Dämpfen) erhaltene native Öl verstanden. Das Öl ist von gelber bis gelbgrüner Farbe mit charakteristischem Geruch und süßlichbitterem Geschmack. Das Öl ist reich an einfach- und mehrfach ungesättigten Fettsäuren insbesondere an ungesättigten Fettsäuren.

In einer bevorzugten Ausführungsform ist daher das Traubenkernöl wie folgt charakterisiert:

| | |
|---|---|
| Dichte (20°C) | 0,913 -0,926 |
| Brechungsindex (20°C) | 1,479 -1,477 |
| Säurezahl | max. 1 |
| Peroxidzahl | max. 5 |
| Jodzahl | 120- 151 |
| Verseifungszahl | 182- 196 |
| Unverseifbare Anteile | max. 1,5 Gew. -% |

Enthaltend eine Fettsäurezusammensetzung, vorzugsweise von (in Gew. %):

| | | |
|---|---|---|
| Myristinsäure | C 14:0 | max. 1,0 % |
| Palmitinsäure | C 16:0 | 3,0 -8,0 % |
| Stearinsäure | C 18:0 | 2,5 -5,0 % |
| Ölsäure | C 18:1 | 15,0- 26,0 % |
| Linolsäure | C 18:2 | 65,0 -75,0 % |
| Linolensäure γ | C 18:3 | max. 2,0 % |
| Arachinsäure | C 20:0 | max. 0,5 % |
| Gadoleinsäure | | max. 0,5 % |

Traubenkernöl ist käuflich erhältlich.

In einer weiteren Ausführungsform der Erfindung sind Zusammensetzungen bevorzugt, die als unverseifbaren Bestandteil eines weiteren von Traubenkernöl verschiedenen Öles (Zusatzstoff), nämlich die unverseifbaren Bestandteile aus der Familie Lauracea, insbesondere der Nutzpflanze Persea (z.B. persea americana, persea gratissima (Avocado)) umfassen (nachstehend auch Avocado-Extrakt). Besonders bevorzugt sind solche unverseifbaren Bestandteile der Persea, die 25 - 30 Gew. -% Phytosterine enthalten. Diese Phytosterine setzen sich hauptsächlich vorteilhaft aus β-Sitosterol und Stigmasterol neben anderen wie z.B. Campesterol und Brassicasterol zusammen. Ein besonders bevorzugter Avocado-Extrakt enthaltend unverseifbare Anteile des Avocadoöles ist das käuflich erhältliche Avocadostearin (Gustav Heess, Stuttgart, Germany).

Durch ihre scheibchenförmige Molekülstruktur begünstigten die Phytosterine eine Filmbildung auf der Haut, erschweren auf diese Weise die Resorption von toxischen Substanzen und sorgen für eine gute Hautfeuchtigkeit durch Verhinderung eines übermäßigen transepidermalen Wasserverlustes. Des weiteren ergänzen sie systemisch die Wirkung des Traubenkernöls in der erfindungsgemäßen Wirkstoffkombination.

In einer weiteren Ausführungsform kann die erfindungsgemäße Wirkstoffkombination in einer kosmetischen Zusammensetzung (synonym: Zubereitung) zur topischen oder dermatologischen Verwendung in Form einer Emulsion, z.B. als Creme, Lotion (Hautmilch) oder Paste sowie eines Badezusatzes (z. B. Ölbad), Duschbades, Peelings erfolgen. Ebenfalls sind wasserfreie Systeme möglich und bevorzugt, z.B. Hautöl oder Ölbad.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Sind die beiden Flüssigkeiten Wasser und Öl und liegen Öltröpfchen fein verteilt in Wasser vor, so handelt es sich um eine Öl-in-Wasser-Emulsion (O/W-Emulsion). Der Grundcharakter einer O/W-Emulsion ist durch das Wasser geprägt. Bei einer Wasser-in-Öl-Emulsion (W/O-Emulsion) handelt es sich um das umgekehrte Prinzip, in dem der Grundcharakter hier durch das Öl bestimmt wird. Weiterhin sind Mischsysteme wie Wasser-in-Öl-in-Wasser-Emulsionen (W/O/W-Emulsion) und Öl-in-Wasser-in-Öl-Emulsionen (O/W/O-Emulsionen) bekannt. Alle genannten Emulsionen sind erfindungsgemäß geeignet.

Zu den erfindungsgemäß geeigneten wasserfreien Systemen gehören reine Ölzubereitungen wie z.B. Hautöle oder Ölbäder. Ebenfalls einsetzbare Pasten enthaltend die erfindungsgemäße Zusammensetzung, zeichnen sich dadurch aus, dass sie aus den gleichen oder ähnlichen Bestandteilen wie eine Emulsion bestehen aber im Wesentlichen wasserfrei sind. Im Rahmen der vorliegenden Erfindung werden die Begriffe Ölphase und Lipidphase synonym verwendet. In einer weiteren bevorzugten Ausführungsform kann die erfindungsgemäße Zusammensetzung als weiteren Bestandteil einen Emulgator beinhalten. In einer ganz bevorzugten Ausführung kann dieser Emulgator ein O/W-Emulgator sein.

Emulgatoren können vorteilhaft gewählt werden aus der Gruppe der nichtionischen, anionischen, kationischen oder amphoteren Emulgatoren.

Als nichtionischen Emulgator können verschiedene Emulgatoren aus den Gruppen der Partialfettsäureester, Fettalkohole, Sterole, Polyethylengylcole wie z.B. ethoxylierte Fettsäuren, ethoxylierte Fettalkohole und ethoxylierte Sorbitanester, Zuckeremulgatoren, Polyglycerinemulgatoren oder Silikonemulgatoren Verwendung finden.

Als anionischen Emulgator können verschiedene Emulgatoren aus den Gruppen der Seifen z. B. Natriumstearat, Fettalkoholsulfate, Mono-, Di- und Trialkylphosphosäureester und deren Ethoxylate, Fettsäure Lactat Ester, Fettsäure Citrat Ester oder Fettsäure Citroglycerinester Verwendung finden.

Als kationische Emulgatoren können beispielsweise quaternäre Ammoniumverbindungen mit einem langkettigen aliphatischen Rest z. B. Distearyldimonium Chloride eingesetzt werden.

Unter den amphoteren Emulgatoren können verschiedene Emulgatoren aus den Gruppen Alkylaminoalkancarbonsäuren, Betaine, Sulfobetaine oder Imidazolinderivate Verwendung finden.

Erfindungsgemäß bevorzugt sind natürlich vorkommende Emulgatoren, zu denen beispielsweise Bienenwachs, Wollwachs, Lecithin und Sterole u.a. gehören die ebenfalls bei der Herstellung einer erfindungsgemäßen Zusammensetzung eingesetzt werden können. In einer bevorzugten Formulierung der erfindungsgemäßen Zusammensetzung können O/W-Emulgatoren ausgewählt werden aus der Gruppe der Pflanzenproteinhydrolysaten und deren Derivate.

Zur Herstellung der erfindungsgemäßen Zusammensetzung lassen sich in vorteilhafter Weise die Ester und Salze von Weizenproteinhydrolysaten sowie Mono und Diester von Partialglyceriden als O/W-Emulgatoren verwenden. Ganz besonders bevorzugt werden Mischungen aus dem Weizenproteinhydrolysat (Potassium Palmitoyl Hydrolyzed Wheat Protein), Glycerylstearat und Cetearylalkohol eingesetzt. Insbesondere eignet sich eine Mischung bestehend aus 10 - 25 Gew.-% Potassium Palmitoyl Hydrolyzed Wheat Protein, 25 - 50 Gew.-% Glycerylstearat und 25 - 50 Gew. -% Cetearylalkohol zur Herstellung der erfindungsgemäßen Zusammensetzung. Eine derartige Kombination ist unter dem Handelsnamen Phytocream erhältlich.

Im Sinne der vorliegenden Erfindung können ferner vorteilhaft Substanzen als Zusatzstoffe enthalten sein, ausgewählt aus der Gruppe der Ester aus gesättigten und/oder ungesättigten, verzweigten und/oder unverzweigten Alkancarbonsäuren und/oder Alkencarbonsäuren mit einer Kettenlange von 3 - 30 Kohlenstoffatomen und gesättigten und/oder ungesättigten, verzweigten und/oder unverzweigten Alkoholen einer Kettenlange von 3 - 30 Kohlenstoffatomen sowie aus der Gruppe der Ester aus aromatischen Carbonsäuren und gesättigten und/oder ungesättigten, verzweigten und/oder unverzweigten Alkoholen einer Kettenlange von 3 bis 30 C-Atomen. Solche Esterole können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononyliso-nonanoat, 2-Ethylhexylpalmitat, 2-Ethylhexyllaurat, 2- Hexyldecylstearat, 2-Octyldodecyl-palmitat, Oleyloleat, Oleylerucat, Erucyloleat, Erucylerucat sowie synthetische, halbsynthetische und natürliche Gemische solcher Ester, wie z.B. Jojobaöl.

Jojobaöl wird aus den reifen Samen des Jojobastrauches (*Simmondsia chinensis*) durch mechanische Pressung und anschließender Raffination gewonnen. Das Öl ist eine klare wasserhelle Flüssigkeit, die fast keinen Geruch aufweist. Das Jojobaöl gleicht in seiner chemischen Struktur einem Wachs, das einer langkettigen einfach ungesättigten Fettsäure aufgebaut ist, insbesondere aus Gadoleinsäure und Erucasäure.

Ferner kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 - 24 C-Atomen, insbesondere 12 - 18 C-Atomen. Die Fettsäuretriglyceride können beispielsweise vorteilhaft gewählt werden aus der Gruppe der synthetischen, halbsynthetischen und natürlichen Öle.

Ebenfalls geeignet ist Erdnussöl, welches aus dem Samen der subtropischen Erdnusspflanze (*Arachis Hypogaea)* durch Pressung und Extraktion gewonnen wird. Anschließend erfolgt die Entschleimung (degumming), Filtration und Raffination (Entsäuern, Bleichen, Dämpfen). Man erhält dadurch ein blassgelbliches Öl mit leicht an die Samen erinnerndem Geruch und Geschmack. Das Öl ist reich an einfach- und mehrfachungesättigten Fettsäuren insbesondere an den beiden essentiellen Fettsäuren Ölsäure und Linolsäure.

Weiterhin geeignet ist Mandelöl, ein kaltgepresstes, raffiniertes, fettes Öl aus den reifen Samen von *Prunus dulcis* (Miller) D.A. Webb var. *dulcis* oder *Prunus dulcis* (Miller) D.A. Webb var. *amara* (D.C.) Buchheim oder aus einer Mischung von beiden. Das Öl ist eine hellgelbe, klare, durchscheinende Flüssigkeit mit schwachem, charakteristischem Geruch und schwachem charakteristischem, süßlichem Geschmack. Das Mandelöl ist reich an einfach- und mehrfach ungesättigten Fettsäuren insbesondere an den beiden essentiellen Fettsäuren Ölsäure und Linolsäure.

Daher betrifft die Erfindung ebenfalls eine erfindungsgemäße Zusammensetzung, die als Zusatzstoffe, beispielsweise Jojobaöl, Olivenöl, Sonnenblumenöl, Sojaöl, Erdnussöl, Rapsöl, Mandelöl, Palmöl, Kokosöl, Palmkernöl oder dergleichen enthält. Ganz besonders bevorzugt sind pflanzliche Öle, die mindestens 6 Gew. -% an mehrfach ungesättigten Fettsäuren aufweisen.

In einer weiteren bevorzugten Ausführungsform enthalten die erfindungsgemäßen Zusammensetzungen mindestens 1 Gew. -%, vorzugsweise 1 - 30 Gew. -%, besonders bevorzugt 2 - 15 Gew. - % oder gar 5-10 Gew. -% eines solchen, mindestens einem weiteren pflanzlichen Öls als Zusatzstoff.

Insbesondere können in den erfindungsgemäßen Zusammensetzungen zusätzlich als Hilfs- oder Zusatzstoff Antioxidantien und/oder Radikalfängern zugesetzt werden. Vorteilhaft werden solche Antioxidantien gewählt aus der Gruppe der lipophilen Systeme beispielsweise: natürliche und synthetische Tocopherole, Nordihydroguajaretsäure, Coniferylbenzoat, Butylhydroxyanisol, Butlyhydroxy-tolul, Gallussäureester und verschiedenen antioxidativen Pflanzen Extrakten. Unter den hydrophilen Systemen sind besonders vorteilhaft anorganischen Schwefelverbindungen, Natriumhydrogensulfit, Cystein oder Ascorbinsäure zu verwenden.

Die erfindungsgemäßen kosmetischen Zusammensetzungen können weiterhin kosmetische Hilfsstoffe enthalten, wie sie üblicherweise in solchen Zusammensetzungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, weichmachende, anfeuchtende und/oder feuchthaltende Substanzen oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere kann die kosmetische Zusammensetzung Mittel zum Peeling der Haut, insbesondere Partikel zum mechanischen Peeling, enthalten. Die Partikel können z. B. aus Polyethylen bestehen. Die Mittel zum Peeling können dabei vorzugsweise im Bereich von 1 bis 20 Gew.-%, vorzugsweise von 3 bis 15 Gew.-% und besonders bevorzugt von 5 bis 10 Gew.-% in der Zusammensetzung enthalten sein.

In einer weiteren besonderen Ausführungsform ist die erfindungsgemäße Zusammensetzung im Wesentlichen aus natürlich vorkommenden Inhaltsstoffen zusammengesetzt, wie oben im Einzelnen genannt.

Darüber hinaus betrifft die Erfindung auch ein kosmetisches Verfahren gekennzeichnet durch die Schritte, dass zunächst eine kosmetische Zusammensetzung enthaltend eine vorbeschriebene Wirkstoffkombination sowie geeignete Hilfs- und Zusatzstoffe in Form eines Badezusatzes oder Duschbades auf zu behandelnden Hautstellen angewendet wird und anschließend eine oder mehrere kosmetische Zusammensetzungen enthaltend eine vorstehend beschriebene Wirkstoffkombination sowie geeignete Zusatz- und Hilfsstoffe in Form einer Creme, Lotion, Paste, Hautöl oder Emulsion, auch in wasserfreier Form, aufgebracht wird. Insbesondere kann diese zweite oder weitere kosmetische Zusammensetzung, aber auch die zuerst angewandte kosmetische Zusammensetzung, Peelingpartikel, insbesondere aus Polyester als Zusatzstoffe enthalten. Die zweite kosmetische Zusammensetzung kann dabei vorzugsweise in die Haut einmassiert werden, wohingegen die erste Zusammensetzung durch Abspülen mit Wasser sowie gegebenenfalls Abtrocknen der entsprechenden Hautstellen zumindest teilweise entfernt wird vor dem zweiten Schritt.

Des weiteren betrifft die Erfindung die Verwendung der erfindungsgemäßen Zusammensetzung zur Prophylaxe trockener oder atopischer Haut und Hautalterungserscheinungen, insbesondere Faltenbildung.

Des Weiteren betrifft die Erfindung die Verwendung der erfindungsgemäßen Wirkstoffkombination oder einer erfindungsgemäßen Zusammensetzung zur Prophylaxe und Behandlung trockener oder atopischer Haut und von Hautalterungserscheinungen insbesondere Faltenbildung sowie die Verwendung der erfindungsgemäßen Wirkstoffkombination oder der erfindungsgemäßen kosmetischen Zusammensetzung zur Prophylaxe und Behandlung von Bindegewebsschwäche und Zellulite.

Nachstehende Beispiele dienen zur näheren Erläuterung der Erfindung, ohne die Erfindung auf diese Beispiele zu beschränken.

Die INCI-Nomenklatur kann zu den vor- und nachstehenden Stoffen entsprechend verwendet werden.

### Beispiele:

### Beispiel 1:

### Zusammensetzung eines Hautöls:

| Gew.-% | | |
|---|---|---|
| 60,190 | Soonnenblumenöl | Gustav Heess GmbH & Co. KG, |
| | | Stuttgart, Deutschland |
| 15,000 | Traubenkernöl | Gustav Heess GmbH & Co. KG, |
| | | Stuttgart, Deutschland |
| 15,000 | Jojobawachs | Gustav Heess GmbH & Co. KG, |
| | | Stuttgart, Deutschland |
| 3,200 | Avocadin | Gustav Heess GmbH & Co. KG, |
| | | Stuttgart, Deutschland |
| 3,000 | Rizinusöl | Henry Lamotte, Bremen, |
| | | Deutschland |
| 2,000 | Tocopherolacetat | DSM Nutrional Products, Basel, |
| | | Schweiz |
| Ad 100 | Parfümöl | Fa. Michael Schmitt, Köln, |
| | | Deutschland |
| 0,500 | Santalbinsäure | Selco GfN, Wald-Michelbach, |
| | | Deutschland |
| 0,100 | Antioxidant | Tocopherol Cognis Deutschland |
| | | GmbH, Düsseldorf, Deutschland |
| 0,060 | Vitamin-A-Palmitat | DSM Nutrional Products, Basel, |
| | | Schweiz |

### Beispiel 2:

### Zusammensetzung einer Lotion (Creme)

| Gew.-% | | |
|---|---|---|
| Ad 100 | Wasser, | |
| | demineralisiert | |
| 21,000 | Traubenkernöl | Gustav Heess GmbH & Co. KG, Stuttgart, |
| | | Deutschland |
| 5,000 | Hydrolite-5 | Symrise GmbH & Co. KG, Holzminden, |
| | | Deutschland |
| 5,000 | Rizinusöl | Henry Lamotte, Bremen, Deutschland |
| 5,000 | Sonnenblumenöl | Gustav Heess GmbH & Co. KG, |
| | | Stuttgart, Deutschland |
| 4,000 | Phytocream | Biesterfeld Spezialchemie GmbH, |
| | | Hamburg, Deutschland |
| | | Staub & Co. Chemiehandels- |
| 3,000 | Glycerol 85% | gesellschaft mbH, Nürnberg, |
| | | Deutschland |
| 3,000 | Sheabutter | Nordmann, Rassmann GmbH&Co. |
| | | Hamburg, Deutschland |
| 2,500 | Avocadin | Gustav Heess GmbH & Co. KG, |
| | | Stuttgart, Deutschland |
| 2,000 | Tocopherolacetat | DSM Nutrional Products, Basel, |
| | | Schweiz |
| 1,500 | Cetearyl Alcohol | Cognis Deutschland GmbH, |
| | | Düsseldorf, Deutschland |
| 0,800 | Parfümöl | Fa. Michael Schmitt, Köln, |
| | | Deutschland |
| 0,080 | Antioxidant | z.B. Tocopherol Cognis Deutschland |
| | | GmbH, Düsseldorf, Deutschland |
| 0,500 | Santalbinsäure | Selco GfN, Wald-Michelbach, |
| | | Deutschland |
| 0,015 | Citronensäure | DSM Nutrional Products, Basel, |
| | | Schweiz |

### Beispiel 3:

### Zusammensetzung eines Ölbades

| Gew.-% | | |
|---|---|---|
| 88,875 | Traubenkernöl | Gustav Heess GmbH & Co. KG, |
| | | Stuttgart, Deutschland |
| 5,000 | Parfümöl | Fa. Michael Schmitt, Köln, |
| | | Deutschland |
| | | Henry Lamotte, Bremen, |
| 3,000 | Rizinusöl | Deutschland |
| | | DSM Nutrional Products, Basel, |
| 2,000 | Tocopherolacetat | Schweiz |
| 1,000 | Arlatone | Unichema Chemie GmbH, Emmerich, |
| | | Deutschland |
| 0,100 | Antioxidant | Tocopherol Cognis Deutschland |
| | | GmbH, Düsseldorf, Deutschland |
| 0,500 | Santalbinsäure | Selco GfN, Wald-Michelbach, |
| | | Deutschland |

### Beispiel 3a:

### Zusammensetzung eines Ölbades

| Gew.-% | | |
|---|---|---|
| Ad 100 | Traubenkernöl Deutschland | Gustav Heess GmbH & Co. KG, |
| | | Stuttgart, |
| 5,00 | Parfümöl | Fa. Michael Schmitt, Köln, |
| | | Deutschland |
| 3,00 | Rizinusöl | Henry Lamotte, Bremen, |
| | | Deutschland |
| 2,00 | Tocopherolacetat | DSM Nutrional Products, Basel, |
| | | Schweiz |
| 1,00 | Arlatone | Unichema Chemie GmbH, Emmerich, |
| | | Deutschland |
| 0,10 | Antioxidant | Tocopherol Cognis Deutschland |
| | | GmbH, Düsseldorf, Deutschland |
| 0,50 | Santalbinsäure | Selco GfN, Wald-Michelbach, |
| | | Deutschland |

### Beispiel 4:

### Zusammensetzung einer Bodybutter

| Gew.-% | | |
|---|---|---|
| ad 100 | Wasser, gereinigt | |
| 17,500 | Traubenkernöl Vitis Vinifera | Jan Dekker GmbH, |
| | (Grape) Seed Oil | Lanqenfeld, Deutschland |
| | Sheabutter | Nordmann, Rassmann GmbH |
| 10,000 | Butyrospermum Parkii (Shea Butter) | & Co., Hamburg, |
| | Fruit | Deutschland |
| 6,000 | Phytocream | Fa. Biesterfeld Wilhelm E. H., Frankfurt, Deutschland |
| | Cetearyl Alcohol, Glyceryl | |
| | Stearate und Potassium Palmitoyl | |
| | Hydrolized Wheat Protein | |
| | Mangokernbutter | Jan Dekker GmbH, Langenfeld, Deutschland |
| 5,000 | Magnifera Indica (Mango) Seed | |
| | Butter | |
| 3,000 | Glycerol 85% | Fa. Gustav Heess, |
| | Glycerin in Wasser | Stuttgart, Deutschland |
| 2,000 | Cetearyl Alcohol (Lanette O) | Cognis Deutschland GmbH, |
| | Cetearyl Alcohol | Düsseldorf, Deutschland |
| 0,600 | Parfum (Fragrance) | Fa. Michael Schmitt, |
| | | Köln, Deutschland |
| 0,500 | Dermosoft Octiol | Dr. Straetmans, Hamburg, |
| | Caprylyl Glycol | Deutschland |
| | Avocadin | Croda GmbH, Nettetal, |
| 0,200 | Persea Gratissima Oil (Avocado) | Deutschland |
| | Unsaponifiables | |
| 0,200 | Ximenoil | Selco, Wald-Michelbach, |
| | Santalbinsäure | Deutschland |
| 0,200 | Polyacrylsäure (Carbopol Ultrez 21) | Gattefossé, Weil am |
| | Acrylates/C10-30 Alkyl Acrylate | Rhein, Deutschland |
| 0,050 | Mischtocopherolkonzentrat 70% | Cognis Deutschland GmbH, |
| | (natürlich) | Düsseldorf, Deutschland |

### Beispiel 5:

### Zusammensetzung eines Bodypeelings

| Gew.-% | | |
|---|---|---|
| ad 100 | Nasser gereinigt | |
| 11,000 | Traubenkernöl | Jan Dekker GmbH, |
| | Vitis Vinifera (Grape) Seed Oil | Langenfeld, Deutschland |
| 10,000 | Polyethylen 617A | Nordmann, Rassmann GmbH & Co., Hamburg, Deutschland |
| | Polyethylen-Peeling-Körner | |
| 6,000 | Rizinusöl | Henry Lamotte Oils GmbH, |
| | Ricinus Communis (Castor) Seed Oil | Bremen, Deutschland |
| 6,000 | Hydrolite-5 | Symrise GmbH & Co. KG, |
| | Pentylene Gycol | Holzminden, Deutschland |
| 4,000 | Glycerol 85% | Fa. Gustav Heess, |
| | Glycerin in Wasser | Stuttqart, Deutschland |
| 2,000 | Jojobawachs | Fa. Gustav Heess, Stuttgart, Deutschland |
| | Simmondsia Chinensis (Jojoba) Seed | |
| | Oil | |
| 1,800 | Aristoflex AVL | |
| | Caprylic/Capric Triglyceride (and) | |
| | Ammonium Acryldimethyltaurate/ VP- | Clariant GmbH, Sulzbach, |
| | Copolymer (and) Trilaureth-4 | Deutschland |
| | Phosphate (and) Polyglyceryl-2- | |
| | Sesquiisostearate | |
| 0,700 | Parfum (Fragrance) | Fa. Michael Schmitt, |
| | | Köln, Deutschland |
| 0,600 | Walocel HM | Degussa (Goldschmidt GmbH), Essen, Deutschland |
| | Hydroxypropyl Methylcellulose | |
| 0,500 | Avocadin | Croda GmbH, Nettetal, Deutschland |
| | Persea Gratissima (Avocado) Oil | |
| | Unsaponifiables | |
| 0,500 | Ximenoil | Selco, Wald-Michelbach, |
| | Santalbinsäure | Deutschland |
| <0,100 | Mischtocopherolkonzentrat 70% | Cognis Deutschland GmbH, |
| | (natürlich) | Düsseldorf, Deutschland |

### Beispiel 6:

### Zusammensetzung eines Duschpeelings

| Gew.-% | | |
|---|---|---|
| ad 100 | Wasser, gereinigt | |
| 15, 000 | Texapon N 70 | Cognis Deutschland GmbH, Düsseldorf, Deutschland |
| | in Natriumlaurethsulfat in | |
| | Wasser | |
| 10,000 | Glycerol 85% | Fa. Gustav Heess, Stuttgart, |
| | Glycerin in Wasser | Deutschland |
| 6,000 | Plantacare 818 | Cognis Deutschland GmbH, |
| | Coco-Glucoside in Wasser | Düsseldorf, Deutschland |
| 5,000 | Tego Betaine F 50 in | Degussa (Goldschmidt GmbH), Essen, Deutschland |
| | Cocamidopropyl Betaine in | |
| | Wasser | |
| 5,000 | Polyethylen 617A | Nordmann, Rassmann GmbH & |
| | Polyethylen-Peeling-Körner | Co., Hamburg, Deutschland |
| 3,000 | Euperlan PK 1200 | Cognis Deutschland GmbH, |
| | | Düsseldorf - Deutschland |
| 2,500 | Arlatone T (V) | Dr. W. Kolb AG, Hedingen, |
| | | Schweiz |
| 2,000 | Lamesoft PO 65 | Cognis Deutschland GmbH, |
| | | Düsseldorf, Deutschland |
| 1,000 | Traubenkernöl | Jan Dekker GmbH, Langenfeld, Deutschland |
| | Vitis Vinifera (Grape) Seed | |
| | Oil | |
| 1,000 | Jojobawachs | Fa. Gustav Heess, Stuttgart, |
| | | Deutschland |
| 0,900 | Polyacrylsäure (Carbopol | |
| | Ultrez 20) | Gattefossé, Weil am Rhein, |
| | Acrylates/C10-30 Alkyl | Deutschland |
| | Acrylate Crosspolymer | |
| 1,250 | Parfum (Fragrance) | Polygon Chemie AG, Olten, Deutschland |
| 0,300 | Walocel HM 4000 PA 2910 | Degussa (Goldschmidt GmbH, |
| | Hydroxypropyl Methylcellulose | Essen, Deutschland |
| 0,100 | Avocadin | Croda GmbH, Nettetal, Deutschland |
| | Persea Gratissima (Avocado) | |
| | Oil Unsaponifiables | |
| 0,100 | Ximenoil | Selco, Wald-Michelbach, |
| | Santalbinsäure | Deutschland |
| < | Mischtocopherolkonzentrat 70% | Cognis Deutschland GmbH, |
| 0,010 | (natürlich) | Düsseldorf, Deutschland |
| auf pH | Natronlauge 50% | Fa. Biesterfeld Wilhelm E.H., |
| 5,5 | | Frankfurt, Deutschland |

### Beispiel 7:

### Zusammensetzung einer Dusche (Duschbades)

| Gew.-% | | |
|---|---|---|
| ad 100 | Wasser, gereinigt | |
| 15,000 | Texapon N 70 | Cognis Deutschland GmbH, |
| | Natriumlaurethsulfat in Wasser | Düsseldorf, Deutschland |
| 10,000 | Glycerol 85% | Fa. Gustav Heess, |
| | Glycerin in Wasser | Stuttqart, Deutschland |
| 6,000 | Plantacare 818 | Cognis Deutschland GmbH, |
| | Coco-Glucoside in Wasser | Düsseldorf, Deutschland |
| 5,000 | Tego Betaine F 50 | Degussa (Goldschmidt GmbH, |
| | Cocamidopropyl Betaine in Wasser | Essen - Deutschland |
| 2,900 | Antil 171 | Degussa (Goldschmidt GmbH, Essen - Deutschland |
| | PEG-18 Glyceryl Oleate/Cocoate | |
| | in Wasser | |
| 2,500 | Arlatone T (V) | Dr. W. Kolb AG, Hedingen, |
| | PEG 40-Sorbitan Peroleat | Schweiz |
| 2,000 | Lamesoft PO 65 | Cognis Deutschland GmbH, Düsseldorf, Deutschland |
| | Glyceryl Oleate und Coco- | |
| | Glucoside in Wasser | |
| 0,630 | Traubenkernöl | Jan Dekker GmbH, |
| | Vitis Vinifera (Grape) Seed Oil | Langenfeld, Deutschland |
| 1,000 | Euperlan PK 1200 | Cognis Deutschland GmbH, Düsseldorf, Deutschland |
| | Coco-Glucoside, Glycol | |
| | Distearate und Glycerin in | |
| | Wasser | |
| 0,900 | Parfum (Fragrance) | Fa. Michael Schmitt, Köln, |
| | | Deutschland |
| 0,100 | Avocadin | Croda GmbH, Nettetal, Deutschland |
| | Persea Gratissima (Avocado) Oil | |
| | Unsaponifiables | |
| 0,100 | Ximenoil | Selco, Wald-Michelbach, |
| | Santalbinsäure | Deutschland |
| < | Mischtocopherolkonzentrat 70% | Cognis Deutschland GmbH, |
| 0,010 | (natürlich) | Düsseldorf, Deutschland |
| auf pH | Natronlauge 50% | Fa. Biesterfeld Wilhelm E. |
| 5,5 | | H., Frankfurt, Deutschland |

### Beispiel 8: Messungen einzelner Parameter

Die Messungen wurden an 34 Personen (weibl.) mit einem Durchschnittsalter 43,7 +/- 7,3 Jahren im Winter (29 Tage) durchgeführt. In den Messungen wurde die Wirksamkeit der Kombination der Produkte *Hautöl (Beispiel 1), Bodylotion (Beispiel* 2) *und Ölbad (Beispiel* 3), der erfindungsgemäßen Wirkstoffkombination untersucht.
Als vierter Faktor wurde eine Massage der behandelten Hautregionen mit einem Sisal-Handschuh durchgeführt.

### a.) Hautstraffung und Geschmeidigkeit der Haut - Cutometer-Test, Cutometrie

Die Hautelastizität wurde mit dem Cutometer SEM 575, Fa. Courage + Khazaka, gemessen. Die Messung werde mit einem Instrument durchgeführt, welches ein Vakuum aufbaut und ein bestimmtes Areal der Haut einsaugt. Der Wert wird optisch wiedergegeben. Die Analyse der aufgezeichneten Messwerte kann die Hautcharakteristiken in Bezug auf Elastizität und Plastizität wiedergeben.

### Auswertung:

| Parameter | Gebiet | Cutometer | | | |
|---|---|---|---|---|---|
| | | Tag 0 | Tag 29 | Unterschied | p-Wert |
| R0 | Rechter Oberschenkel | 0,46 | 0,43 | | |
| | Linker Oberschenkel | 0,46 | 0,43 | | |
| | Mittel | 0,46 | 0,43 | -0,03 | 0,02 |
| R1 | Rechter Oberschenkel | 0.05 | 0,09 | | |
| | Linker Oberschenkel | 0,07 | 0,09 | | |
| | Mittel | 0,06 | 0,09 | 0,03 | <0,001 |

| | | | | | |
|---|---|---|---|---|---|
| p-Wert signifikant, da p<0,05 | | | | | |

Ergebnis: Eine signifikante Erhöhung der Hautstraffheit (R0-Wert) und der Geschmeidigkeit bzw. Elastizität (R1-Wert) ist erfolgt.

### b.) Durchblutungsförderung - Laser-Doppler-Imager (LDI) Messung

Die Laser-Doppler-Flußmessung (Laser-Doppler-Imager Firma Perimed AB, Järfälla Schweden), erlaubt einfach zu handhabende, eingriffslose Realzeit-Messungen des lokalen Gewebeblutflusses. Graphisch oder digital können der mittlere Fluß, die Konzentration und die mittlere Geschwindigkeit der roten Blutkörperchen angezeigt werden.

### Auswertung:

| | LDI | | | | |
|---|---|---|---|---|---|
| | Tag 1 | Tag 1 nach 5 Minuten | Unterschied | Tag 1 nach 60 Minuten | Unterschied |
| Rechter Oberschenkel | 0,40 | 0,45 | | 0,44 | |
| Linker Oberschenkel | 0,41 | 0,46 | | 0,43 | |
| Mittel | 0,41 | 0,46 | 0,05 | 0,44 | 0,04 |
| p-Wert | | < 0,001 | | < 0,001 | |

| | | | | | |
|---|---|---|---|---|---|
| Steigerung der Durchblutung signifikant, da p < 0,05 | | | | | |

Ergebnis: Nach der Anwendung ist eine signifikante durchblutungsfördernde Wirkung erfolgt.

### c.) Hautfeuchtigkeit - Corneometer-Test, Corneometrie

Mit dem Corneometer CM 825, Fa. Courage + Khazaka, wird die Stratum corneum-Hydratation (Hornschichtfeuchtigkeit) gemessen. Die Hornschichtfeuchtigkeit ist ein wesentlicher Parameter, der Rückschlüsse auf die epidermale Barrierefunktion zuläßt und es erlaubt, sowohl den schädigenden Einfluß von Noxen, die auf die Hautoberfläche einwirken, als auch den hydratisierenden Nutzeffekt von Externa quantitativ zu erfassen. Beim Corneometerprinzip wird durch eine Kapazitätsmessung die Hautfeuchtigkeit der "äußeren Schicht" der Oberhaut (Stratum corneum) bestimmt. Diesem Prinzip liegt die Tatsache der unterschiedlichen Dielektrizitätskonstanten von Wasser und anderen Stoffen zugrunde. Ein entsprechend geformter Meßkondensator reagiert auf die in sein Meßvolumen eingebrachten Proben mit unterschiedlichen Kapazitätsänderungen, die vom Gerät vollautomatisch erfaßt und ausgewertet werden. Die mit Spezialglas beschichtete aktive Sonde wird auf die zu messende Hautstelle gedrückt und nach 1 Sekunde erscheint auf der Anzeige der Corneometermeßwert, also der Grad der Feuchtigkeit auf der Hautoberfläche. Durch eine besondere Konstruktion wird sichergestellt, dass die aktive Stirnfläche der Sonde jeweils mit konstanter Kraft auch auf alle unzulänglichen Hautstellen gedrückt wird.
Der Anzeigewert des Corneometers gibt den Grad der Feuchtigkeit auf der Hautoberfläche an, z.B. vor und nach Behandlungen der Haut mit kosmetischen oder pharmazeutischen Erzeugnissen, d.h. das Gerät zeigt den Zustand bzw. die Veränderung der Feuchtigkeit der Hautoberfläche an.

### Auswertung:

| | Corneometer | | | |
|---|---|---|---|---|
| | Tag 0 | Tag 29 | Unterschied | p-Wert |
| Linker Oberschenkel | 35,96 | 39,51 | | |
| Rechter Oberschenkel | 35,03 | 39, 10 | | |
| Mittel | 35,50 | 39,30 | 3,81 | 0,029 |

Steigerung der Hautfeuchte um 3,81; Signifikant, da p<0,5 Ergebnis: Eine signifikante Erhöhung der Hautfeuchte ist erfolgt.

### d.) Cellulite-Wirksamkeit

Visuelle Überprüfung samt Oberschenkel-Umfangsmessung. Auswertung:

| Scala 1-5 | Probanden |
|---|---|
| 0 Verschlechterung | 0 |
| 1 keine Verbesserung | 5 |
| 2 leichte Verbesserung | 17 |
| 3 moderate Verbesserung | 10 |
| 4 starke Verbesserung | 2 |
| Mittel der Verbesserung | 2,26 |

Ergebnis: Die Auswertung der subjektiven Einschätzungen der Probanden ergibt, dass ein curativer Effekt bei der Cellulite-Behandlung erfolgt ist.

### Beispiel 9:

### Weitere vergleichende LDI-Messungen:

Jede Untersuchung e) bis h) wurde an jeweils vier weiblichen Probanden mit einem Durchschnittsalter von 45,1 +/- 8,8 Jahren durchgeführt. Zur Anwendung wurde eine Konzentrationsreihe von Hautölen (jeweils 100µl) mit unterschiedlicher Zusammensetzung gebracht. Das Öl wurde auf eine Hautfläche von 3x15 cm auf dem Oberarm mittels leichter Massage verteilt. Die Messungen wurden vor und direkt nach dem Auftragen sowie nach 30 und 60 Minuten durchgeführt, und in einem Unterbereich der behandelten Testbereiche der Größe 2x2 cm mit einem Laser-Doppler-Imager, Firma Perimed AB, Järfälla (SE) bestimmt.

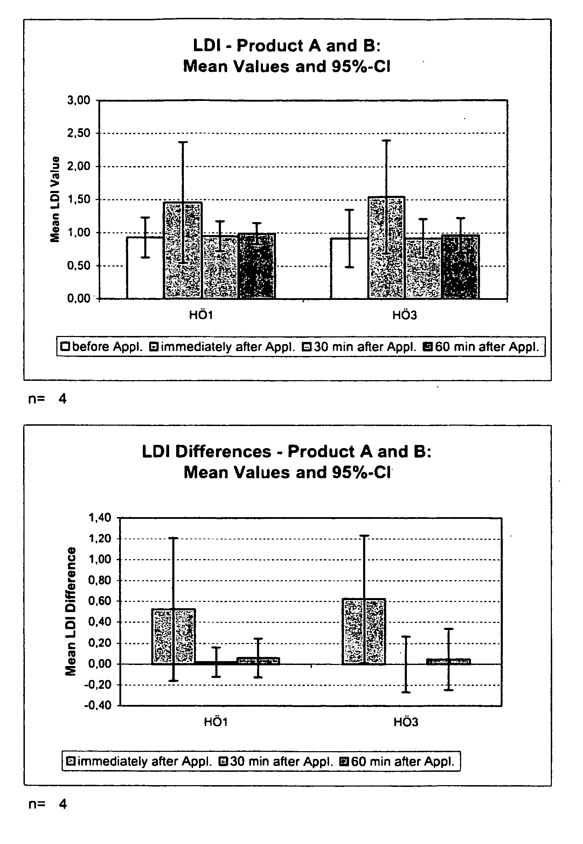

Bei e) wurde Traubenkernöl gegen Traubenkernöl plus 0,5 Gew.-% Santalbinsäure(Produkt A (HÖ1) und B (HÖ3)) gemessen. Das Ergebnis ist der Figur 1 zu entnehmen.
Santalbinsäure unterstützt vorteilhaft den Massageeffekt des Traubenkernöls.

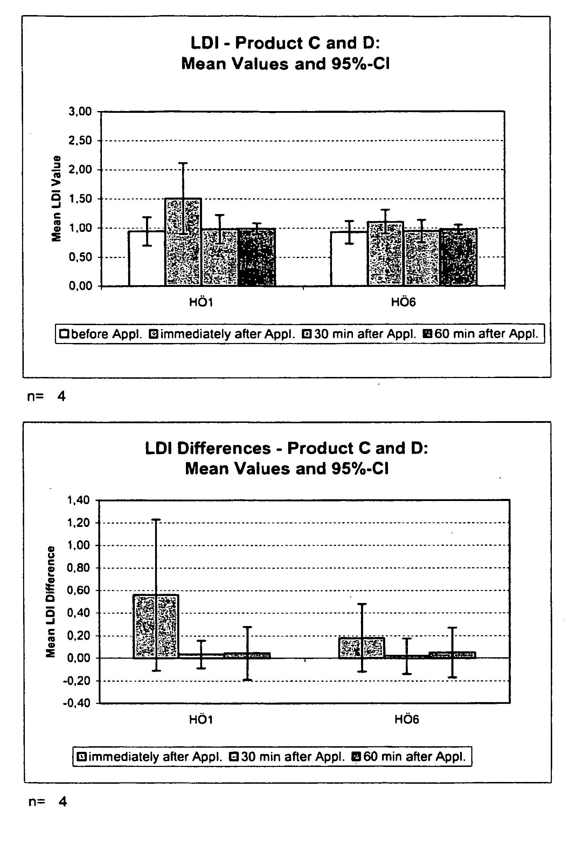

Bei f) wurde Traubenkernöl gegen Isopropylmyristat (IPM) (Produkt C (HÖ1) und D (HÖ6)) gemessen. Das Ergebnis ist der Figur 2 zu entnehmen.
Traubenkernöl führt zu einer Verbesserung der Hautdurchblutung gegenüber IPM. Dies ist insbesondere auf die hervorragenden Gleitmitteleigenschaften des Traubenkernöls zurückzuführen.

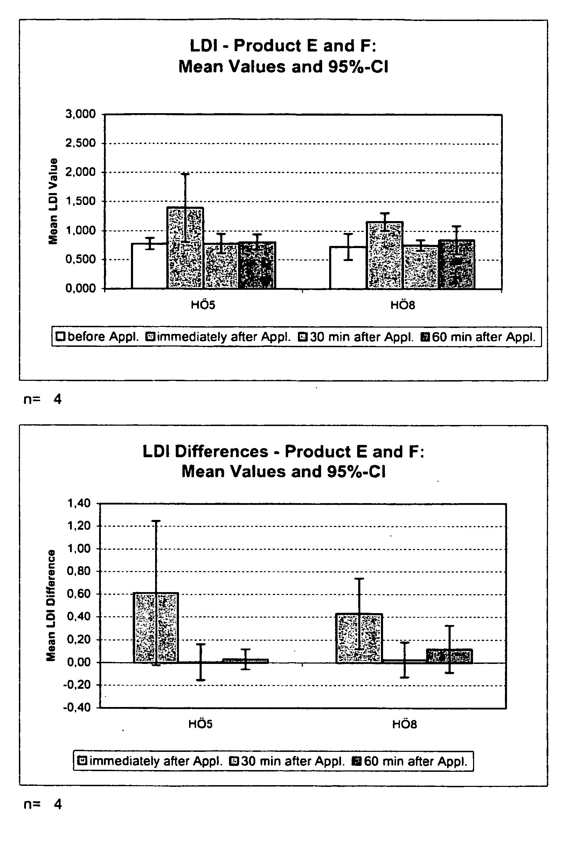

Bei g) wurde Traubenkernöl plus 0,5 Gew.-% Santalbinsäure und 3,2 Gew.-% Avocadin gegen IPM plus 0,5 Gew.-% Santalbinsäure (Proukt E (HÖ5) und F (HÖ8)) gemessen. Das Ergebnis ist der Figur 3 zu entnehmen.
Avocadin führt zu einer Steigerung der Durchblutung.

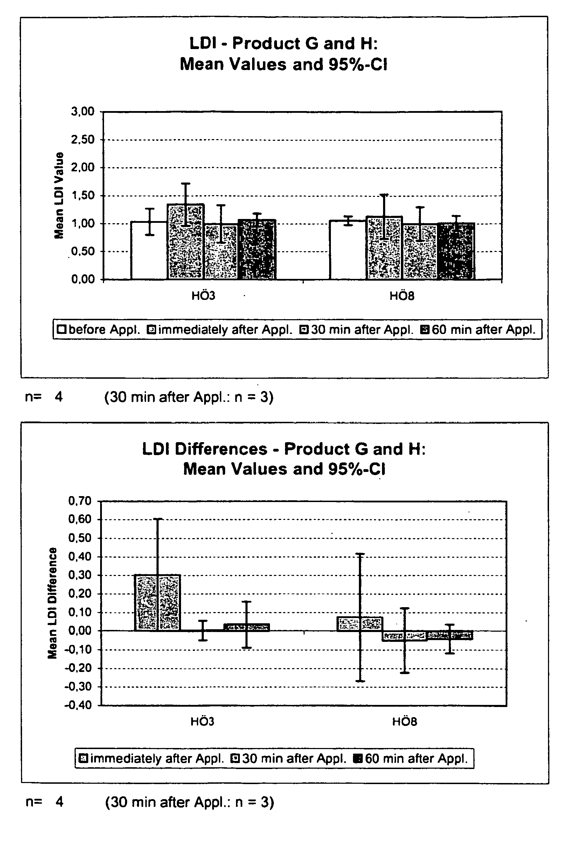

Bei h) wurde Traubenkernöl plus 0,5 Gew.-% Santalbinsäure gegen IPM plus 0,5 Gew.-% Santalbinsäure (Produkt G (HÖ3) und H (HÖ8)) gemessen. Das Ergebnis ist der Figur 4 zu entnehmen. Die erfindungsgemäße Wirkstoffkombination erweist sich überlegen und führt zu einer verbesserten Durchblutung.

## Patentansprüche

1. Wirkstoffkombination zur topischen oder dermatologischen Verwendung enthaltend Traubenkernöl als einen primären Wirkstoff und Verbindungen als zweiten Wirkstoff bestehend aus mindestens 12 Kohlenstoffatomen gemäß Formel (I): 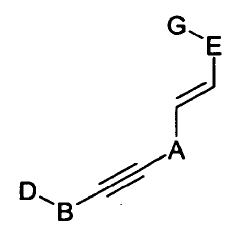 mit A eine Bindung oder (CH₂)ₙ mit n= 1-6
und B und E unabhängig voneinander C₂-C₁₂ Alkylen, C₂-C₁₂ Alkenylen,
D und G beide COR oder zumindest D oder G COR, wobei der jeweils andere H, CH₃ ist und R unabhängig voneinander für D oder G ausgewählt ist aus der Gruppe OH, CH₃, C₂H₅, NH₂ oder deren Salze davon.

2. Wirkstoffkombination nach Anspruch 1, **dadurch gekennzeichnet, dass** die Verbindungen gemäß Formel I mit A= eine Bindung oder A = CH₂ und n=1
und / oder
B und E mit (CH₂)ₙ und n= 4-8 und D oder G = COR, wobei der jeweils andere CH₃ ist und R OH oder CH₃ ist.

3. Wirkstoffkombination nach Anspruch 1 oder 2, **dadurch gekennzeichnet, dass** der zweite Wirkstoff Santalbinsäure oder ein Ester davon ist.

4. Wirkstoffkombination nach einem der Ansprüche 1 bis 3, **dadurch gekennzeichnet, dass** die Wirkstoffkombination folgende Verhältnisse aufweist: Traubenkernöl in Gew % 1-99%, vorzugsweise 1-90 %, 1-50%, 1-20% und eine Verbindung bestehend aus mindestens 12 C-Atomen gemäß Formel (I) in Gew % 0,01 - 5, vorzugsweise Gew % 0,02-1 oder Gew % 0,02 - 0,5 und weitere Hilfs- und Zusatzstoffe.

5. Wirkstoffkombination nach einem der Ansprüche 1 bis 4, **dadurch gekennzeichnet, dass** ein weiterer Zusatzstoff die unverseifbaren Bestandteile eines pflanzlichen Öls aus einem Extrakt der Nutzpflanze Persea enthält.

6. Kosmetische Zusammensetzung enthaltend eine Wirkstoffkombination nach einem der Ansprüche 1 bis 5 und geeignete Hilfs- und Zusatzstoffe.

7. Kosmetische Zusammensetzung enthaltend eine Wirkstoffkombination nach Anspruch 6, in Form eines Badezusatzes oder Duschbades.

8. Kosmetische Zusammensetzung enthaltend eine Wirkstoffkombination nach Anspruch 6, in Form einer Creme, Lotion, Paste, Hautöl oder Emulsion.

9. Kosmetische Zusammensetzung enthaltend eine Wirkstoffkombination nach Anspruch 6, in wasserfreier Form, insbesondere Hautöl.

10. Kosmetische Zusammensetzung nach einem der Ansprüche 6 bis 9, **dadurch gekennzeichnet, dass** als Zusatzstoff Peeling-Partikel, insbesondere im Bereich von 1 bis 20 Gew.-%, vorzugsweise 3 bis 15 Gew.-% und vorzugsweise 5 bis 10 Gew.-%, enthalten sind.

11. Kosmetische Zusammensetzung nach Anspruch 10, **dadurch gekennzeichnet, dass** die Peeling-Partikel aus Polyethylen bestehen.

12. Kosmetische Zusammensetzung nach einem der Ansprüche 6 bis 11, **dadurch gekennzeichnet, dass** ein oder mehrere Zusatzstoffe ausgewählt sind aus der Gruppe Jojobaöl, Olivenöl, Sonnenblumenöl, Sojaöl, Erdnussöl, Rapsöl, Mandelöl, Palmöl, Kokosöl, Palmkernöl.

13. Kosmetische Zusammensetzung nach einem der Ansprüche 10 bis 12, **dadurch gekennzeichnet, dass** die Zusatzstoffe zu mindestens 1 Gew. -%, 1 - 30 Gew. -% oder vorzugsweise 2 - 15 Gew.-% enthalten sind.

14. Kosmetische Zusammensetzung nach einem der Ansprüche 6 bis 13, **dadurch gekennzeichnet, dass** ein O/W-Emulgator, insbesondere ein Pflanzenproteinhydrolysat, enthalten ist.

15. kosmetischen Verfahren zur Behandlung der Haut **gekennzeichnet, durch** die Schritte, dass zunächst eine kosmetische Zusammensetzung gemäß der Ansprüche 7 sowie 10 bis 14, soweit auf Anspruch 7 rückbezogen, auf einem Körper zumindest teilweise angewandt wird und anschließend eine oder mehrere kosmetische Zusammensetzungen nach Anspruch 8 oder 9 sowie 10 bis 14, soweit auf Anspruch 8 oder 9 rückbezogen, auf diese Körperstellen aufgetragen wird.

16. Verwendung einer Wirkstoffkombination oder einer kosmetischen Zusammensetzung nach einem der Ansprüche 1 bis 14 zur kosmetischen Hautpflege, nämlich Prophylaxe und Behandlung von trockener Haut und Hautalterungserscheinungen, insbesondere Faltenbildung.

17. Verwendung einer Wirkstoffkombination oder einer kosmetischen Zusammensetzung nach einem der Ansprüche 1 bis 14 zur kosmetischen Hautpflege, nämlich zur Prophylaxe und Behandlung von Bindegewebsschwäche und Cellulite.

18. Verwendung einer Wirkstoffkombination oder einer kosmetischen Zusammensetzung nach einem der Ansprüche 1 bis 14 zur Herstellung eines Mittels zur Prophylaxe und Behandlung von atopischer Haut.

## Claims

1. Active ingredient combination for topical or dermatological use, comprising grape seed oil as a primary active ingredient and, as a second active ingredient, compounds containing at least 12 carbon atoms, according to Formula (I): 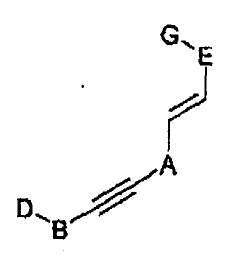 wherein A is a bond or (CH₂)ₙ wherein n = 1-6,
and B and E, independently of one another, are C₂-C₁₂ alkyls or C₂-C₁₂ alkenyls,
D and G are both COR, or at least D or G is COR, the other one in each case being H or CT₃, and R being selected, for D or G independently of one another, from the group consisting of OH, CH₃, C₂H₅, NH₂ or the salts thereof.

2. Active ingredient combination according to claim 1, **characterised in that** compounds according to Formula I wherein
A = a bond or A = CH₂ and n = 1
and/or
B and E are (CH₂)ₙ and n = 4-8, and D or G = COR, the other one in each case being CH₃, and R being OH or CH₃.

3. Active ingredient combination according to either claim 1 or claim 2, **characterised in that** the second active ingredient is santalbic acid or an ester thereof.

4. Active ingredient combination according to any one of claims 1 to 3, **characterised in that** the active ingredient combination has the following proportions: grape seed oil at 1-99 % by weight, preferably 1-90 %, 1-50 %, 1-20%, and a compound containing at least 12 C-atoms in accordance with Formula (I) at 0.01-5 % by weight, preferably 0.02-1 % by weight or 0.02-0.5 % by weight, and further auxiliary excipients and additives.

5. Active ingredient combination according to any one of claims 1 to 4, **characterised in that** a further additive contains the unsaponifiable component of a plant oil from an extract of the useful plant Persea.

6. Cosmetic composition containing an active ingredient combination according to any one of claims 1 to 5 and appropriate excipients and additives.

7. Cosmetic composition containing an active ingredient combination according to claim 6, in the form of a bath additive or shower gel.

8. Cosmetic composition containing an active ingredient combination according to claim 6, in the form of a cream, lotion, paste, skin oil or emulsion.

9. Cosmetic composition containing an active ingredient combination according to claim 6, in an anhydrous form, in particular skin oil.

10. Cosmetic composition according to any one of claims 6 to 9, **characterised in that** peeling particles are included as an additive, especially at about 1 to 20 % by weight, preferably 3 to 15 % by weight and preferably 5 to 10 % by weight.

11. Cosmetic composition according to claim 10, **characterised in that** the peeling particles consist of polyethylene.

12. Cosmetic composition according to any one of claims 6 to 11, **characterised in that** one or more additives are selected from the group consisting of jojoba oil, olive oil, sunflower oil, soya oil, peanut oil, rapeseed oil, almond oil, palm oil, coconut oil and palm seed oil.

13. Cosmetic composition according to any one of claims 10 to 12, **characterised in that** the additives are contained at at least 1 % by weight, 1-30 % by weight, or preferably 2-15 % by weight.

14. Cosmetic composition according to any one of claims 6 to 13, **characterised in that** an O/W emulsifier, in particular a plant protein hydrolysate, is included.

15. Cosmetic process for skin treatment, **characterised by** the steps of: initially applying a cosmetic composition according to claim 7, and claims 10 to 14 in so far as these refer back to claim 7, to a body, at least in part; and subsequently applying one or more cosmetic compositions according to either claim 8 or claim 9, and claims 10 to 14 in so far as these refer back to claim 8 or 9, to this part of the body.

16. Use of an active ingredient combination or a cosmetic composition according to any one of claims 1 to 14 for cosmetic skin-care, in particular the prophylaxis and treatment of dry skin and the appearance of skin ageing, in particular the formation of wrinkles.

17. Use of an active ingredient combination or a cosmetic composition according to any one of claims 1 to 14 for cosmetic skin-care, in particular the prophylaxis and treatment of connective tissue weakness and cellulite.

18. Use of an active ingredient combination or a cosmetic composition according to any one of claims 1 to 14 to produce a means for the prophylaxis and treatment of atopic skin.

## Revendications

1. Combinaison d'agents actifs à usage topique ou dermatologique contenant de l'huile de pépins de raisin comme agent actif primaire et, comme second agent actif, des composés constitués d'au moins 12 atomes de carbone suivant la formule (I) : 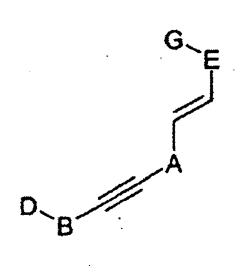 A représentant une liaison ou (CH₂)ₙ avec n = 1-6
et B et E représentant, indépendamment l'un de l'autre, un alkylène en C₂-C₁₂, un alcénylène en C₂-C₁₂,
D et G représentant tous les deux COR ou au moins D ou G représentant COR, l'autre représentant alors H, CH₃ et R étant choisi indépendamment pour D ou G dans le groupe OH, CH₃, C₂H₅, NH₂ ou leurs sels.

2. Combinaison d'agents actifs selon la revendication 1, **caractérisée par** les composés selon la formule I avec A = une liaison ou A = CH₂ et n = 1 et/ou B et E représentant (CH₂)ₙ avec n = 4-8 et D ou G = COR, l'autre représentant alors CH₃ et R représentant OH ou CH₃.

3. Combinaison d'agents actifs selon la revendication 1 ou 2, **caractérisée en ce que** le second agent actif est un acide santalique ou un ester de celui-ci.

4. Combinaison d'agents actifs selon l'une des revendications 1 à 3, **caractérisée en ce que** la combinaison d'agents actifs présente les rapports suivants : de 1 à 99 % en poids d'huile de pépins de raison, de préférence de 1 à 90 %, de 1 à 50 %, de 1 à 20 % en poids, et de 0,01 à 5 % en poids d'un composé constitué d'au moins 12 atomes de C selon la formule (I), de préférence de 0,02 à 1 % en poids ou de 0,02 à 0,5 % en poids, et d'autres agents auxiliaires et adjuvants.

5. Combinaison d'agents actifs selon l'une des revendications 1 à 4, **caractérisée en ce qu'**un autre adjuvant contient les constituants insaponifiables d'une huile végétale à partir d'un extrait de la plante utile Persea.

6. Composition cosmétique contenant une combinaison d'agents actifs selon l'une des revendications 1 à 5 et des agents auxiliaires et adjuvants appropriés.

7. Composition cosmétique contenant une combinaison d'agents actifs selon la revendication 6, sous la forme d'un adjuvant de bain ou bain-douche.

8. Composition cosmétique contenant une combinaison d'agents actifs selon la revendication 6, sous la forme d'une crème, lotion, pâte, huile corporelle ou émulsion.

9. Composition cosmétique contenant une combinaison d'agents actifs selon la revendication 6, sous forme anhydre, en particulier d'huile corporelle.

10. Composition cosmétique selon l'une des revendications 6 à 9, **caractérisée en ce qu'**elle contient en tant qu'adjuvant des particules de peeling, en particulier dans la proportion de 1 à 20 % en poids, de préférence de 3 à 15 % en poids et de préférence de 5 à 10 % en poids.

11. Composition cosmétique selon la revendication 10, **caractérisée en ce que** les particules de peeling sont en polyéthylène.

12. Composition cosmétique selon l'une des revendications 6 à 11, **caractérisée en ce qu'**un ou plusieurs adjuvants sont choisis parmi le groupe constitué de l'huile de jujube, huile d'olive, huile de tournesol, huile de soja, huile de noisette, huile de colza, huile d'amande, huile de palme, huile de coco, huile de palmiste.

13. Composition cosmétique selon l'une des revendications 10 à 12, **caractérisée en ce qu'**elle contient les adjuvants à hauteur d'au moins 1 % en poids, de 1 à 30 % en poids ou de préférence de 2 à 15 % en poids.

14. Composition cosmétique selon l'une des revendications 6 à 13, **caractérisée en ce qu'**elle contient un émulsifiant H/E, en particulier un hydrolysat de protéines végétales.

15. Procédé cosmétique pour le soin de la peau, **caractérisé par** les étapes consistant à appliquer dans un premier temps, au moins en partie sur un corps, une composition cosmétique selon les revendications 7 ainsi que 10 à 14, dans la mesure où celles-ci se réfèrent à la revendication 7, et à appliquer ensuite sur ces parties du corps une ou plusieurs compositions cosmétiques selon la revendication 8 ou 9 ainsi que 10 à 14, dans la mesure où celles-ci se réfèrent à la revendication 8 ou 9.

16. Utilisation d'une combinaison d'agents actifs ou d'une composition cosmétique selon l'une des revendications 1 à 14 pour le soin cosmétique de la peau, à savoir pour la prophylaxie et le traitement des peaux sèches et des phénomènes d'altération de la peau, en particulier la formation de rides.

17. Utilisation d'une combinaison d'agents actifs ou d'une composition cosmétique selon l'une des revendications 1 à 14 pour le soin cosmétique de la peau, à savoir pour la prophylaxie et le traitement des faiblesses du tissu conjonctif et de la cellulite.

18. Utilisation d'une combinaison d'agents actifs ou d'une composition cosmétique selon l'une des revendications 1 à 14 pour la fabrication d'un produit destiné à la prophylaxie et au traitement des peaux atopiques.
